(19) Europäisches Patentamt
European Patent Office
Office européen des brevets

(11) EP 3 097 129 B1

(12) FASCICULE DE BREVET EUROPEEN

(45) Date de publication et mention de la délivrance du brevet:
**27.06.2018 Bulletin 2018/26**

(21) Numéro de dépôt: **15705640.9**

(22) Date de dépôt: **20.01.2015**

(51) Int Cl.:
***C08F 293/00*** (2006.01) ***C08F 2/22*** (2006.01)
***A61K 8/90*** (2006.01) ***B82Y 40/00*** (2011.01)
***C09K 8/52*** (2006.01) ***C09D 153/00*** (2006.01)
***A61K 8/02*** (2006.01) ***A61Q 19/00*** (2006.01)
***B82Y 30/00*** (2011.01) ***C09K 8/536*** (2006.01)
***C09D 7/40*** (2018.01)

(86) Numéro de dépôt international:
**PCT/FR2015/050134**

(87) Numéro de publication internationale:
**WO 2015/110750 (30.07.2015 Gazette 2015/30)**

# (54) LATEX DE PARTICULES SPHÉRIQUES AUX PROPRIÉTES DE VIEILLISSEMENT THERMIQUE AMÉLIORÉES

LATEX AUS SPHÄRISCHEN PARTIKELN MIT VERBESSERTEN WÄRMEALTERUNGSEIGENSCHAFTEN

LATEX OF SPHERICAL PARTICLES HAVING IMPROVED THERMAL AGEING PROPERTIES

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **21.01.2014 FR 1450444**

(43) Date de publication de la demande:
**30.11.2016 Bulletin 2016/48**

(73) Titulaire: **Arkema France**
**92700 Colombes (FR)**

(72) Inventeur: **INOUBLI, Rabi**
**F-64000 Pau (FR)**

(56) Documents cités:
**WO-A1-02/077075**
**WO-A1-2008/046156**
**WO-A1-2010/096867**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 3 097 129 B1

## Description

La présente invention concerne des particules polymériques ayant une forme sphérique constituées de copolymères à blocs et leurs propriétés de résistance au vieillissement thermique.

La synthèse de latex de particules sphériques de tailles nanométriques, (méth)acryliques ou autres, est connue.

La publication de G. Delaittre et al. Chem. Commun. 2009, 2887-2889 décrit la préparation de vésicules de copolymères à blocs amphiphiles PNaA-b-P4VP. Ceux-ci sont obtenus en milieu aqueux, à pH 11, en une seule étape, par PRC en émulsion; la réaction de polymérisation de la 4VP est amorcée à l'aide d'une macroalcoxyamine de polyacrylate de sodium-SG1 soluble dans l'eau. Les copolymères obtenus s'assemblent pour former des agrégats, l'extension des chaînes s'effectuant du côté des chaînes hydrophobes. Les observations au microscope électronique en transmission (TEM) ont montré que les particules polymériques formées ont des tailles en dessous du micromètre et que leurs formes sont variables, allant des particules sphériques (60 nm) à des micelles vermiformes (60 nm de largeur), puis à des vésicules sphériques et vésicules allongées ou vésicules à compartiments multiples, comme montré dans la figure 3 de ce document.

La publication de S. Boissé et al. dans Chem. Commun. 2010, 46, 1950-1952 expose la préparation de nanofibres constituées de copolymères à blocs amphiphiles par polymérisation radicalaire par transfert réversible par addition fragmentation (RAFT). Le styrène est polymérisé dans l'eau en présence d'un agent RAFT macromoléculaire (ou macroagent RAFT) hydrophile. Lorsque l'homopolymère de l'acide acrylique (AA) ou du méthyl éther acrylate de poly(ethylene glycol) (PEGA) est employé comme macroagent RAFT, les copolymères à blocs obtenus se présentent sous forme de micelles sphériques (comme montré dans la figure 1 de l'article cité, (1) et (9) respectivement).

La publication de Y. Li et S.P. Armes dans Angew. Chem. Int. Ed. 2010, 49, 4042-4046 décrit la synthèse de latex de particules sphériques de tailles nanométriques (20 à 100 nm) par auto-assemblage d'un copolymère dibloc PGMA-PHPMA synthétisé par voie RAFT.

La publication de S. Sugihara et al. dans Soft Matter, 2011, 7, 10787 décrit des nanoparticules formées de copolymères dibloc réticulés, lesdits copolymères consistant en des chaînes de PMPC et un coeur de PHPMA réticulé au moyen d'EDGMA. Des structures sphériques sont obtenues avec des taux nuls ou très faibles d'EDGMA.

Le document WO 2010/096867 décrit des particules polymériques hétérogènes comprenant un polymère formé à la surface d'un autre polymère. Ces particules sont obtenues au moyen d'un procédé comprenant deux étapes de polymérisation :

- dans une première étape on polymérise au moins un monomère à insaturation éthylénique en présence d'un agent RAFT pour obtenir une dispersion aqueuse de grains de particules polymères, qui sont ensuite réticulées ;
- dans une deuxième étape on polymérise un monomère à insaturation éthylénique à la surface desdites particules réticulées, pour obtenir un polymère qui aura une structure différente de celle desdits grains.

Les polymères ainsi obtenus sont utilisés dans des compositions aqueuses et organiques pour revêtements, cosmétiques, adhésifs et autres.

Aucun de ces documents ne décrit les propriétés de résistance au vieillissement desdites particules sphériques.

Il a maintenant été trouvé que, de manière surprenante, certaines micelles sphériques ont la propriété de présenter une résistance accrue au vieillissement, notamment au vieillissement thermique, en milieu dispersé si leur coeurs sont réticulés chimiquement.

L'invention a pour objet l'utilisation d'un latex de particules polymériques sphériques réticulées constituées de copolymères à blocs synthétisés par polymérisation radicalaire contrôlée en émulsion, pour améliorer la résistance au vieillissement thermique d'une composition aqueuse ou organique. De manière caractéristique, ces particules polymériques se présentent sous forme de sphères ayant un diamètre de 10 à 100 nm et sont obtenues par l'auto-assemblage desdits copolymères à blocs.

Selon un mode de réalisation, la synthèse desdites particules est effectuée à partir d'au moins un monomère hydrophobe et d'un agent réticulant en présence d'un macroamorceur vivant dérivé d'un nitroxyde, caractérisé en ce que :

- lesdites particules sphériques sont obtenues en milieu aqueux directement lors de la synthèse desdits copolymères à blocs effectuée en chauffant le milieu de réaction à une température de 60 à 120°C,
- ledit macroamorceur est hydrosoluble,
- le pourcentage de la masse molaire du macroamorceur hydrosoluble dans le copolymère à blocs final est compris entre 10 et 50%, et en ce que
- le taux de conversion du monomère hydrophobe est d'au moins 50%.

Cette méthode directe de préparation de particules sphériques réticulées ne nécessite pas l'emploi de cosolvant organique.

Dans le cadre de la présente invention, le terme « particules sphériques » correspond à des auto-assemblages de macromolécules amphiphiles qui, lorsqu'elles sont en suspension dans l'eau (autrement dit, lorsqu'elles forment une dispersion aqueuse), prennent spontanément la forme d'une sphère dont le coeur est constitué des éléments hydrophobes et la surface des éléments hydrophiles desdites macromolécules. Ces particules sphériques sont observables au microscope électronique en transmission (TEM). Les images de microscopie montrent des sphères dont le diamètre est supérieur ou égal à 10 nm.

Selon un autre mode de réalisation, la synthèse desdites particules sphériques est effectuée par polymérisation radicalaire par transfert réversible par addition fragmentation (RAFT) dans l'eau en présence d'un agent RAFT macromoléculaire (ou macroagent RAFT) hydrophile.

Lesdites compositions aqueuses dont la résistance au vieillissement, notamment au vieillissement thermique est améliorée, trouvent des applications en tant qu'additifs de solutions aqueuses ou organiques.

L'invention et les avantages qu'elle procure seront mieux compris à la lumière de la description détaillée qui va suivre et des figures 1 et 2 annexées qui illustrent les particules sphériques selon l'invention vues en microscopie électronique en transmission (TEM).

Il a maintenant été trouvé qu'une composition comprenant lesdites particules polymériques sphériques réticulées, présente une résistance accrue au vieillissement, notamment au vieillissement thermique, ce qui lui permet de pouvoir être utilisée plus longtemps et dans une plage de températures plus large que les compositions de l'art antérieur.

Avantageusement, la réticulation chimique au coeur de la micelle sphérique permet de rendre le latex totalement immiscible, même dans les solvants qui dissolvent l'ensemble du polymère. De plus, cette réticulation permet au latex de particules sphériques en milieu dispersé (milieu aqueux par exemple), de ne pas se dégrader après avoir subi un vieillissement thermique de plusieurs jours à des températures supérieures à 100°C. La même structure obtenue par le même auto-assemblage mais qui ne bénéficie par d'une réticulation chimique du coeur va voir sa structure totalement dégradée après le même vieillissement thermique.

A cet effet, l'invention a pour objet l'utilisation d'un latex de particules polymériques sphériques réticulées constituées de copolymères à blocs synthétisés par polymérisation radicalaire contrôlée en émulsion, pour améliorer la résistance au vieillissement thermique de la composition. De manière caractéristique, ces particules polymériques se présentent sous forme de sphères ayant un diamètre compris entre 10 et 100nm. Par le terme «latex» on comprend ici une phase continue, aqueuse ou organique, dans laquelle sont dispersées les particules polymériques sphériques constituées de copolymères à blocs. Ces particules sont constituées de macromolécules amphiphiles qui ont la capacité de s'auto-assembler.

Selon un mode de réalisation, la synthèse desdites particules est effectuée à partir d'au moins un monomère hydrophobe et d'un agent réticulant en présence d'un macroamorceur vivant dérivé d'un nitroxyde.

De manière caractéristique, lesdites particules sphériques réticulées sont obtenues en milieu aqueux de synthèse desdits copolymères à blocs effectuée en chauffant le milieu de réaction à une température de 60 à 120°C, avec un pourcentage de la masse molaire du macroamorceur hydrophile dans le copolymère à blocs final compris entre 10 et 50%, le taux de conversion du monomère hydrophobe et d'agent réticulant étant d'au moins 50%. L'agent réticulant est introduit avantageusement dans le milieu réactionnel à une teneur d'au moins 1% et de préférence entre 5 et 15% en poids par rapport au poids de monomère hydrophobe. Le pH initial du milieu aqueux peut varier entre 5 et 10. Cette méthode directe de préparation de particules sphériques réticulées ne nécessite pas l'emploi de co-solvant organique.

Par « macroamorceur vivant » on entend un polymère comprenant au moins une extrémité apte à être réengagée dans une réaction de polymérisation par ajout de monomères à une température et pression appropriées. Avantageusement, ledit macroamorceur est préparé par PRC. Par « macroamorceur hydrosoluble » on entend un polymère soluble dans l'eau comportant à son extrémité une fonction réactive capable de réamorcer une polymérisation radicalaire. Ce macroamorceur est composé majoritairement de monomères hydrophiles, c'est à dire des monomères présentant une ou plusieurs fonctions aptes à établir des liaisons hydrogène avec l'eau. Dans le cas de la polymérisation d'un monomère hydrophobe, on formera un copolymère amphiphile dont le bloc hydrophile sera constitué par le macroamorceur alors que le bloc hydrophobe sera issu de la polymérisation du (des) monomère(s) hydrophobes. Selon une variante de réalisation, ledit macroamorceur hydrosoluble préformé est ajouté au milieu de réaction comprenant au moins un monomère hydrophobe.

Selon une autre variante à l'intérieur du premier mode de réalisation, ledit macroamorceur hydrosoluble est synthétisé dans le milieu de réaction aqueux en une étape préliminaire, sans isolement du macroamorceur formé ni élimination des éventuels monomères hydrophiles résiduels Cette deuxième variante est une polymérisation « one-pot ».

Les monomères hydrophobes peuvent être choisis parmi :

- les monomères vinylaromatiques tels que le styrène ou les styrènes substitués,
- les acrylates d'alkyle, de cycloalkyle ou d'aryle tels que l'acrylate de méthyle, d'éthyle, de butyle, d'éthyl-2 hexyle ou de phényle,
- les méthacrylates d'alkyle, de cycloalkyle, d'alkényle ou d'aryle tels que le méthacrylate de méthyle, de butyle, de

lauryle, de cyclohexyle, d'allyle, d'éthyl-2 hexyle ou de phényle,

- et la vinylpyridine.

Ces monomères hydrophobes sont ajoutés au milieu de réaction, qui comprend majoritairement de l'eau.

L'agent réticulant employé est un comonomère réticulant différent des monomères hydrophobes précités.

Par comonomère réticulant, on entend un monomère qui, de par sa réactivité avec les autres monomères présents dans le milieu de polymérisation, est capable de générer un réseau tridimensionnel covalent. D'un point de vue chimique, un comonomère réticulant comprend généralement au moins deux fonctions polymérisables éthyléniques, qui en réagissant, sont susceptibles de créer des ponts entre plusieurs chaînes polymériques.

Ces comonomères réticulants peuvent être susceptibles de réagir avec les monomères hydrophobes insaturés lors de la synthèse desdites particules.

Parmi les comonomères réticulants, on peut citer les divinylbenzènes, les trivinylbenzènes, les (méth)acrylates allyliques, les (méth)acrylates de diallylmaléatepolyol tels que les tri(méth)acrylates de triméthylolpropane, les di(méth)acrylates d'alkylèneglycol qui ont de 2 à 10 atomes de carbone dans la chaîne carbonée tels que les di(méth)acrylates d'éthylèneglycol, les di(méth)acrylates de 1,4-butanediol, les di(méth)acrylates de 1,6-hexanediol, les N,N'-alkylène bisacrylamides, tels que le N,N'-méthylène bisacrylamide. De préférence, on utilisera comme agent réticulant le divinylbenzène ou un diméthacrylate.

La mise en oeuvre dudit procédé permet d'obtenir des particules polymériques sphériques réticulées dans lesquelles le taux massique de la partie hydrophile composant le copolymère à bloc est inférieur à 50%.

De manière caractéristique, les particules sphériques réticulées selon l'invention présentent un pourcentage de la masse molaire du macroamorceur hydrophile dans le copolymère à blocs final compris entre 10 et 50%. De manière préférée, le pourcentage de la masse molaire du macroamorceur hydrosoluble dans le copolymère à blocs final est compris entre 10 et 30%.

Telles qu'observées par TEM, les particules sphériques réticulées selon l'invention se présentent sous forme de sphères ; leur diamètre est constant sur toute leur longueur et est supérieur ou égal à 10 nm. Les particules sphériques selon l'invention voient leurs forme et structure se maintenir en milieu dispersé, indépendamment de leur concentration dans le milieu et/ou des variations de pH ou de salinité de celui-ci.

Selon un deuxième mode de réalisation, la synthèse desdites particules sphériques réticulées est effectuée par polymérisation radicalaire par transfert réversible par addition fragmentation (RAFT) dans l'eau en présence d'un agent RAFT macromoléculaire (ou macroagent RAFT) hydrophile.

L'intégrité structurelle des micelles sphériques selon l'invention ne se trouve pas affectée après un vieillissement thermique de plusieurs jours à des températures supérieures à 100°C en milieu aqueux.

Les compositions visées par la présente invention sont obtenues par l'ajout desdites particules polymériques sphériques réticulées à une solution aqueuse ou organique à un taux massique de minimum 50 ppm. Lesdites compositions dont la résistance au vieillissement, notamment au vieillissement thermique est améliorée, sont particulièrement adaptées à l'utilisation en tant qu'additif dans les fluides destinés à l'industrie de l'extraction pétrolière (agent anti-dépôt). D'autres applications de ces compositions visent le domaine cosmétique, celui des peintures, de la papeterie et des épaississants.

L'invention va maintenant être décrite à l'aide des exemples suivants donnés à titre illustratif et non limitatif.

**Exemple 1.** Préparation du macroamorceur poly(acide méthacrylique-co-styrène sulfonate de sodium) in situ pour le procédé d'obtention de micelles sphériques en une unique étape (EG232)

Cet exemple illustre la préparation d'un copolymère vivant poly(acide méthacrylique-co-styrène sulfonate de sodium), utilisé comme macroamorceur, agent de contrôle et stabilisant, en pied de cuve, pour la synthèse de nanoparticules sous formes de micelles sphériques de copolymères à blocs poly(acide méthacrylique-co-styrène sulfonate de sodium)-*b*-poly(méthacrylate de *n*-butyle-*co*-styrène). Le copolymère amphiphile est synthétisé en une seule étape.

Les conditions de synthèse du macroamorceur peuvent être variées (durée de la polymérisation, teneur en styrène sulfonate de sodium, concentration et pH) pour adapter et faire varier la composition du macroamorceur.

Pour ce faire, un mélange contenant 14,8715 g d'acide méthacrylique (1,79 $mol.L_{aq}^{-1}$ ou 1,55 $mol.L^{-1}$), 3,2713 g de styrène sulfonate de sodium (1,48 $\times$ $10^{-1}$ $mol.L_{aq}^{-1}$ ou 1,28 $\times$ $10^{-1}$ $mol.L^{-1}$ soit $f_{0,SS}$ = 0,076 ; $f_{0,SS} = n_{SS}/(n_{SS} + n_{MAA})$), 0,4101 g de $Na_2CO_3$ (4,01 $\times 10^{-2}$ $mol.L_{aq}^{-1}$ ou 3,48 $\times 10^{-2}$ $mol.L^{-1}$) et 89,1 g d'eau déionisée est dégazé à température ambiante par un bullage d'azote pendant 15 min. En parallèle, 0,7163 g (1,95 $\times 10^{-2}$ $mol.L_{aq}^{-1}$ ou 1,69 $\times 10^{-2}$ $mol.L^{-1}$) de l'alcoxyamine BlocBuilder®-MA est solubilisé dans 7,5394 g de soude 0,4M (1,6 équivalent par rapport aux unités acide méthacrylique du BlocBuilder®-MA) et dégazé pendant 5 minutes.

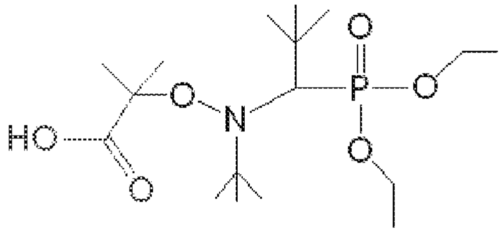

(a)

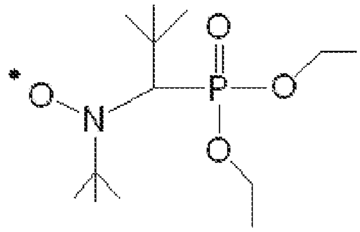

(b)

Schéma de l'amorceur BlocBuilder®-MA (a) et du nitroxyde SG1 (b).

La solution de BlocBuilder®-MA est introduite dans le réacteur à température ambiante sous une agitation à 250 rpm. La solution de monomères est ensuite introduite lentement dans le réacteur. Le réacteur est soumis à une pression de 1,1 bar en azote et toujours sous agitation. Le temps t=0 est déclenché lorsque la température atteint 50 °C. La température du milieu réactionnel atteint 65 °C au bout de 15 minutes.

Pendant cette réaction, dans un erlenmeyer sont introduits 15,09 g de méthacrylate de n-butyle et 1,66 g de styrène (taux de solide = 27,2%) et le mélange est dégazé par un bullage d'azote à température ambiante pendant 10 minutes.

Après 15 minutes de synthèse du macroamorceur du type poly(acide méthacrylique-co-styrène sulfonate de sodium)-SG1, le $t_0$ de la polymérisation en émulsion est déclenché lorsque le second milieu de réaction contenant les monomères hydrophobes est introduit à pression ambiante puis une pression de 3 bar en azote et une agitation à 250 rpm. Le réacteur est maintenu à 90°C tout le long de la polymérisation.

Des prélèvements sont réalisés à intervalles réguliers afin de déterminer la cinétique de polymérisation par gravimétrie (mesure d'extrait sec).

Le tableau 1 et la figure 1 annexée présentent les caractéristiques des latex et particules prélevés à partir de la seconde étape de la synthèse des nanoparticules.

**Tableau 1**

| Temps (h) | Conversion (%) | $M_{n, exp}{}^{a}$ g.mol$^{-1}$ | $M_n$, théo$^b$ g.mol$^{-1}$ | $I_p{}^a$ | pH | Dz$^c$ (nm) | $\Sigma^d$ |
|---|---|---|---|---|---|---|---|
| 0,03 | 14,63 | - | 5617 | - | - | - | - |
| 0,25 | 61,61 | - | 12854 | - | - | - | - |
| 0,50 | 75,05 | - | 14924 | - | - | - | - |
| 0,75 | 83,66 | - | 16249 | - | - | - | - |
| 1,00 | 87,91 | - | 16904 | - | - | - | - |
| 1,25 | 91,83 | - | 17508 | - | - | - | - |
| 1,50 | 93,76 | - | 17806 | - | - | - | - |
| 1,75 | 94,04 | - | 18019 | - | - | - | - |
| 2,80 | 95,15 | - | 18013 | - | - | - | - |
| 3,30 | 95,10 | - | 18767 | - | 4,41 | 48,9 | 0,177 |

Le latex obtenu en fin de polymérisation est translucide et est un peu visqueux (taux de solide élevé).

Evaluation des caractéristiques colloïdales du latex par diffusion dynamique de la lumière : diamètre moyen des particules, distribution en taille des particules.

(a) indice de polydispersité déterminé par chromatographie d'exclusion stérique dans le DMF avec 1g.L$^{-1}$ de LiBr, avec une calibration au polyméthacrylate de méthyle, après méthylation des unités acide méthacryliques en unités méthacrylate de méthyle après purification;

(b)

$$M_{n\ théo} = M_{n\ macro} + [(1-0,27) \times m_{AMA} + (1-0,54) \times m_{SS} + m_{MABu} + m_S] / n_{BlocBuilder®-MA}] \times \text{conversion massique avec } M_{n\ macro} = M_{BlocBuilder®-MA} + (0,27 \times m_{AMA} + 0,54 \times m_{SS})/n_{BlocBuilder®-MA}$$

$M_{n\ théo} = M_{n\ macro} + [(1-0,27) \times m_{AMA} + (1-0,54) \times m_{SS} + m_{MABu} + ms] / n_{BlocBuilder®-MA}] \times$ conversion massique avec $M_{n\ macro} = M_{BlocBuilder®-MA} + (0,27 \times m_{AMA} + 0,54 \times m_{SS})/n_{BlocBuilder®-MA}$

(c) Diamètre moyen en intensité des particules ;

(d) Polydispersité des tailles de particules de latex.

Les mesures (c, d) sont faites par diffusion dynamique de la lumière à 25°C et avec un mode de calcul multi-angle. L'appareil est un Zetasizer Nano Series (NanoZS Zen3600) de Malvern Instrument et le logiciel c'est Zetasizer 6.2. Le NanoZS est calibré avec des latex étalon de PS dans l'eau (taille des particules 200+-6nm). Avant les mesures les échantillons sont dilués avec de l'eau désionisée.

Les imageries sont réalisées par Microscopie Electronique à Transmission TEM (logiciel ImageJ). Ce microscope est de type JEOL 100 Cx II à 100 keV équipé avec une caméra haute résolution CCD camera Keen View de SIS.

**Exemple 2.** Préparation du macroamorceur poly(acide méthacrylique-co-styrène sulfonate de sodium) in situ pour le procédé d'obtention de micelles sphériques réticulées en une unique étape (EG236)

Cet exemple illustre la préparation d'un copolymère vivant poly(acide méthacrylique-co-styrène sulfonate de sodium), utilisé comme macroamorceur, agent de contrôle et stabilisant, en pied de cuve, pour la synthèse de nanoparticules sous formes de micelles sphériques réticulées de copolymères à blocs poly(acide méthacrylique-co-styrène sulfonate de sodium)-*b*-poly(méthacrylate de *n*-butyle-*co*-styrène). Le copolymère amphiphile est synthétisé en une seule étape.

Les conditions de synthèse du macroamorceur peuvent être variées (durée de la polymérisation, teneur en styrène sulfonate de sodium, concentration et pH) pour adapter et faire varier la composition du macroamorceur.

Pour ce faire, un mélange contenant 12,1514 g d'acide méthacrylique (1,51 $mol.L_{aq}^{-1}$ ou 1,34 $mol.L^{-1}$), 2,6705 g de styrène sulfonate de sodium ($1,24 \times 10^{-1}$ $mol.L_{aq}^{-1}$ ou $1,10 \times 10^{-1}$ $mol.L^{-1}$ soit $f_{0,SS} = 0,076$ ; $f_{0,SS} = n_{SS}/(n_{SS} + n_{MAA})$), 0,3922 g de $Na_2CO_3$ ($3,95 \times 10^{-2}$ $mol.L_{aq}^{-1}$ ou $3,50 \times 10^{-2}$ $mol.L^{-1}$) et 87,7 g d'eau déionisée est dégazé à température ambiante par un bullage d'azote pendant 15 min. En parallèle, 0,5847 g ($1,64 \times 10^{-2}$ $mol.L_{aq}^{-1}$ ou $1,45 \times 10^{-2}$ $mol.L^{-1}$) de l'alcoxyamine BlocBuilder®-MA est solubilisé dans 6,1513 g de soude 0,4M (1,6 équivalent par rapport aux unités acide méthacrylique du BlocBuilder®-MA) et dégazé pendant 5 minutes.

La solution de BlocBuilder®-MA est introduite dans le réacteur à température ambiante sous une agitation à 250 rpm. La solution de monomères est ensuite introduite lentement dans le réacteur. Le réacteur est soumis à une pression de 1,1 bar en azote et toujours sous agitation. Le temps t=0 est déclenché lorsque la température atteint 50 °C. La température du milieu réactionnel atteint 65 °C au bout de 15 minutes.

Pendant cette réaction, dans un erlenmeyer sont introduits 12,00 g de méthacrylate de n-butyle et 1,35 g de styrène (taux de solide = 23,8%) et le mélange est dégazé par un bullage d'azote à température ambiante pendant 10 minutes.

Après 15 minutes de synthèse du macroamorceur du type poly(acide méthacrylique-co-styrène sulfonate de sodium)-SG1, le $t_0$ de la polymérisation en émulsion est déclenché lorsque le second milieu de réaction contenant les monomères hydrophobes est introduit à pression ambiante. Une pression de 3 bar en azote et une agitation à 250 rpm sont appliquées. Le réacteur est maintenu à 90°C tout le long de la polymérisation.

Au bout de 45 minutes de polymérisation des monomères hydrophobes, 0,412 g d'éthylène glycol diméthacrylate ($f_{0,EGDMA} = 0,021$ mol) ($f_{0,EGDMA} = n_{EGMDA}/(n_{EGMDA} + n_{MABu} + n_{Sty})$ (taux de solide = 24,0%) est introduit dans le réacteur pour réticuler les sphères après leur formation.

En parallèle de la réaction, 0,051 g de HSA (formaldehydesulfoxylate de sodium) est solubilisé dans 1,9497 g d'eau déionisée, et 0,0454 g de tBHP (hydroperoxyde de *tert*-butyle, 70wt%H2O) est dilué dans 1,9569 g d'eau déionisée ($f_{0,tBHP} = 0,0035$ mol) ($f_{0,tBHP} = n_{tBHP}/n_{tBHP} + n_{EGDMA} + n_{MABu} + n_{Sty}$), soit $n_{tBHP}/n_{HSA} = 1,07$. A 172 minutes, le température du milieu réactionnel est fixée à 70 °C et la solution de HSA est introduite, puis 1 mL d'eau déionisée est introduite pour rincer le conduit. A 183 minutes, la solution de tBHP est introduite, puis 1 mL d'eau déionisée est introduit pour rincer le conduit. La cuisson est effectuée pendant 1h30.

Des prélèvements sont réalisés à intervalles réguliers afin de :

- déterminer la cinétique de polymérisation par gravimétrie (mesure d'extrait sec) ;
- évaluer les caractéristiques colloïdales du latex par diffusion de la lumière : diamètre moyen des particules, distribution en taille des particules (polydispersité).

Le tableau 2 ci-dessous et la figure 2 annexée présentent les caractéristiques des latex et particules prélevés à partir de la seconde étape de la synthèse des nanoparticules.

**Tableau 2**

| Temps (h) | Conversion (%) | $M_{n,exp}$[a] g.mol$^{-1}$ | $M_n$, théo[b] g.mol$^{-1}$ | $I_p$[a] | pH | Dz[c] (nm) | Σ[d] |
|---|---|---|---|---|---|---|---|
| 0,25 | 0,4777 | - | 3365 | - | - | - | - |
| 0,72 | 0,7669 | - | 10625 | - | - | 33,23 | 0,174 |
| 1,25 | 0,8685 | - | 15021 | - | - | - | - |
| 1,75 | 0,8848 | - | 16565 | - | - | - | - |
| 2,32 | 0,8919 | - | 16812 | - | - | - | - |
| 2,75 | 0,8941 | - | 16920 | - | - | - | - |
| 4,55 | 0,9127 | - | 16953 | - | 4,69 | 49,07 | 0,263 |

(b) $M_{n\ théo} = M_{n\ macro} + [(1-0,27) \times m_{AMA} + (1-0,54) \text{ X } m_{SS} + m_{MABu} + ms] / n_{BlocBuilder®-MA}] \times$ conversion massique avec $M_{n\ macro} = M_{BlocBuilder®-MA} + (0,27 \times m_{AMA} + 0,54 \times m_{SS})/n_{BlocBuilder®-MA}$
(c) Diamètre moyen en intensité des particules ;
(d) Polydispersité des tailles de particules de latex.

Le latex obtenu en fin de polymérisation est translucide et très légèrement visqueux. Evaluation des caractéristiques colloïdales du latex par diffusion dynamique de la lumière : diamètre moyen des particules, distribution en taille des particules.

Le réticulant est ajouté à 77% de conversion pour permettre l'auto-assemblage des particules sphériques et éviter l'absence de coagulum lorsqu'on ajoute trop tôt l'EGDMA.

Abréviations :

PRC - polymérisation radicalaire contrôlée
P4VP - poly(4-vinylpyridine)
PNaA - poly(acrylate de sodium)
SG1 -N-tertiobutyl-N-[1-diethylphosphono-(2,2-diméthylpropyl)]
S ou Sty - styrene
SS - styrène sulfonate de sodium
AA - acide acrylique
PEGA - méthyl éther acrylate de poly(ethylene glycol)
PGMA - poly(glycérol monométhacrylate)
PHPMA - poly(hydroxypropyle méthacrylate)
PMPC - poly(2-méthacryloyloxyéthyl phosphorylcholine)
EDGMA - éthylène glycol diméthacrylate
TEM - microscopie électronique en transmission
RAFT - polymérisation par addition fragmentation (« Reversible Addition Fragmentation Chain Transfer »)
MAA - acide méthacrylique
DMSO - dimethylsulfoxyde
DMF - diméthylformamide
Rpm - rotations par minute
$f_{0,sty}$ - fraction molaire initiale de styrène dans le mélange des monomères
$f_{0,SS}$ - fraction molaire initiale de styrène dans le mélange des monomères
$f_{0,DVP}$ - fraction molaire initiale de divinylbenzène dans le mélange des monomères BlocBuilder®-MA - (*N*-(2-methylpropyl)-*N*-(1-diethylphosphono-2,2-dimethylpropyl)-*O*-(2-carboxylprop-2-yl) hydroxylamine

## Revendications

1. Utilisation d'un latex de particules polymériques sphériques réticulées pour améliorer la résistance au vieillissement thermique d'une composition aqueuse ou organique, lesdites particules ayant un diamètre de 10 à 100 nm et étant constituées de copolymères à blocs synthétisés par polymérisation radicalaire contrôlée en émulsion, ledit latex étant obtenu par l'auto-assemblage desdits copolymères à blocs.

2. Utilisation selon la revendication 1 dans laquelle lesdites particules sont synthétisées à partir d'au moins un monomère hydrophobe et d'un agent réticulant en présence d'un macroamorceur vivant dérivé d'un nitroxyde, dans les conditions suivantes :

   - lesdites particules sphériques réticulées sont obtenues en milieu aqueux lors de la synthèse desdits copolymères à blocs effectuée en chauffant le milieu de réaction à une température de 60 à 120°C,
   - ledit macroamorceur est hydrosoluble,
   - le pourcentage de la masse molaire du macroamorceur hydrosoluble dans le copolymère à blocs final est compris entre 10 et 50%, et en ce que
   - le taux de conversion du monomère hydrophobe est d'au moins 50%.

3. Utilisation selon l'une des revendications 1 à 2 dans laquelle le monomère hydrophobe est choisi parmi les monomères vinylaromatiques tels que le styrène ou les styrènes substitués, les acrylates d'alkyle, de cycloalkyle ou d'aryle tels que l'acrylate de méthyle, d'éthyle, de butyle, d'éthylhexyle ou de phényle, les méthacrylates d'alkyle, de cycloalkyle, d'alkényle ou d'aryle tels que le méthacrylate de méthyle, de butyle, de lauryle, de cyclohexyle, d'allyle ou de phényle et la vinylpyridine.

4. Utilisation selon l'une des revendications 1 à 3 dans laquelle le pourcentage de la masse molaire du macroamorceur hydrosoluble dans le copolymère à blocs final est compris entre 10 et 30%.

5. Utilisation selon l'une des revendications 1 à 4 dans laquelle le taux massique de la partie hydrophile composant le copolymère à bloc final est inférieur à 50%.

6. Utilisation selon l'une des revendications 1 à 5 dans ledit comonomère réticulant est choisi parmi les divinylbenzènes, les trivinylbenzènes, les (méth)acrylates allyliques, les (méth)acrylates de diallylmaléatepolyol et les di(méth)acrylates d'alkylèneglycol qui ont de 2 à 10 atomes de carbone dans la chaîne carbonée.

7. Utilisation selon l'une des revendications précédentes dans laquelle l'agent réticulant est introduit dans le milieu réactionnel à une teneur d'au moins 1% et de préférence entre 5 et 15% en poids par rapport au poids de monomère hydrophobe.

8. Utilisation selon l'une des revendications précédentes dans laquelle ladite composition est obtenue par l'ajout desdites particules polymériques sphériques à une solution aqueuse ou organique à un taux massique de minimum 50 ppm.

9. Utilisation selon la revendication 8 dans laquelle ladite composition est utilisée en tant qu'agent anti-dépôt lors de l'extraction d'hydrocarbures.

10. Utilisation selon l'une des revendications 1 à 8 dans laquelle ladite composition est destinée à la préparation de peintures.

11. Utilisation selon l'une des revendications 1 à 8 dans laquelle ladite composition est une composition cosmétique.

## Patentansprüche

1. Verwendung eines Latex von vernetzten kugelförmigen Polymerteilchen zur Verbesserung der Wärmealterungsbeständigkeit einer wässrigen oder organischen Zusammensetzung, wobei die Teilchen einen Durchmesser von 10 bis 100 nm aufweisen und aus durch kontrollierte radikalische Emulsionspolymerisation synthetisierten Blockcopolymeren bestehen, wobei der Latex durch Selbstassemblierung der Blockcopolymere erhalten wird.

2. Verwendung nach Anspruch 1, wobei die Teilchen aus mindestens einem hydrophoben Monomer und einem Vernetzungsmittel in Gegenwart eines lebenden Makroinitiators, der sich von einem Nitroxid ableitet, unter den folgenden Bedingungen synthetisiert werden:

- die vernetzten kugelförmigen Teilchen werden in wässrigem Medium bei der Synthese der Blockcopolymere, die durch Erhitzen des Reaktionsmediums auf eine Temperatur von 60 bis 120 °C durchgeführt wird, erhalten,
- der Makroinitiator ist wasserlöslich,
- der Molmasse-Prozentanteil des wasserlöslichen Makroinitiators in dem fertigen Blockcopolymer liegt zwischen 10 und 50 %, und dadurch, dass
- der Umsatzgrad des hydrophoben Monomers mindestens 50 % beträgt.

3. Verwendung nach einem der Ansprüche 1 bis 2, wobei das hydrophobe Monomer aus vinylaromatischen Monomeren wie Styrol oder substituierten Styrolen, Alkyl-, Cycloalkyl- oder Arylacrylaten wie Methyl-, Ethyl-, Butyl-, Ethylhexyl- oder Phenylacrylat, Alkyl-, Cycloalkyl-, Alkenyl- oder Arylmethacrylaten wie Methyl-, Butyl-, Lauryl-, Cyclohexyl-, Allyl- oder Phenylmethacrylat und Vinylpyridin ausgewählt ist.

4. Verwendung nach einem der Ansprüche 1 bis 3, wobei der Molmasse-Prozentanteil des wasserlöslichen Makroinitiators in dem fertigen Blockcopolymer zwischen 10 und 30 % liegt.

5. Verwendung nach einem der Ansprüche 1 bis 4, wobei der Massenanteil des hydrophilen Teils, aus dem das fertige Blockcopolymer aufgebaut ist, weniger als 50 % beträgt.

6. Verwendung nach einem der Ansprüche 1 bis 5, wobei das vernetzende Comonomer aus Divinylbenzolen, Trivinylbenzolen, Allyl(meth)acrylaten, Diallyl-maleatpolyol(meth)acrylaten und Alkylenglykol-di(meth)acrylaten mit 2 bis 10 Kohlenstoffatomen in der Kohlenstoffkette ausgewählt ist.

7. Verwendung nach einem der vorhergehenden Ansprüche, wobei das Vernetzungsmittel in einem Gehalt von mindestens 1 Gew.-% und vorzugsweise zwischen 5 und 15 Gew.-%, bezogen auf das Gewicht von hydrophobem Monomer, in das Reaktionsmedium eingetragen wird.

8. Verwendung nach einem der vorhergehenden Ansprüche, wobei die Zusammensetzung durch Zugeben der kugelförmigen Polymerteilchen zu einer wässrigen oder organischen Lösung in einem minimalen Massenanteil von 50 ppm erhalten wird.

9. Verwendung nach Anspruch 8, wobei die Zusammensetzung als Antiablagerungsmittel bei der Förderung von Kohlenwasserstoffen verwendet wird.

10. Verwendung nach einem der Ansprüche 1 bis 8, wobei die Zusammensetzung zur Herstellung von Anstrichmitteln bestimmt ist.

11. Verwendung nach einem der Ansprüche 1 bis 8, wobei es sich bei der Zusammensetzung um eine kosmetische Zusammensetzung handelt.

## Claims

1. Use of a latex of crosslinked spherical polymer particles for improving the thermal ageing resistance of an aqueous or organic composition, said particles having a diameter of from 10 to 100 nm and being constituted of block copolymers synthesized by controlled radical emulsion polymerization, said latex being obtained by self-assembly of said block copolymers.

2. Use according to Claim 1, in which said particles are synthesized using at least one hydrophobic monomer and a crosslinking agent in the presence of a living macroinitiator derived from a nitroxide, under the following conditions:

- said crosslinked spherical particles are obtained in aqueous medium during the synthesis of said block copolymers, performed by heating the reaction medium to a temperature of from 60 to 120°C,
- said macroinitiator is water-soluble,
- the molar mass percentage of the water-soluble macroinitiator in the final block copolymer is between 10%

and 50%, and in that
- the degree of conversion of the hydrophobic monomer is at least 50%.

**3.** Use according to either of Claims 1 and 2, in which the hydrophobic monomer is chosen from vinylaromatic monomers such as styrene or substituted styrenes, alkyl, cycloalkyl or aryl acrylates such as methyl, ethyl, butyl, ethylhexyl or phenyl acrylate, alkyl, cycloalkyl, alkenyl or aryl methacrylates such as methyl, butyl, lauryl, cyclohexyl, allyl or phenyl methacrylate, and vinylpyridine.

**4.** Use according to one of Claims 1 to 3, in which the molar mass percentage of the water-soluble macroinitiator in the final block copolymer is between 10% and 30%.

**5.** Use according to one of Claims 1 to 4, in which the mass content of the hydrophilic part of which the final block copolymer is composed is less than 50%.

**6.** Use according to one of Claims 1 to 5, in which said crosslinking comonomer is chosen from divinylbenzenes, trivinylbenzenes, allyl (meth)acrylates, diallyl maleate polyol (meth)acrylates and alkylene glycol di(meth)acrylates containing from 2 to 10 carbon atoms in the carbon chain.

**7.** Use according to one of the preceding claims, in which the crosslinking agent is introduced into the reaction medium to a content of at least 1% and preferably between 5% and 15% by weight relative to the weight of hydrophobic monomer.

**8.** Use according to one of the preceding claims, in which said composition is obtained by adding said spherical polymer particles to an aqueous or organic solution to a minimum mass content of 50 ppm.

**9.** Use according to Claim 8, in which said composition is used as an antideposition agent during oil extraction.

**10.** Use according to one of Claims 1 to 8, in which said composition is intended for preparing paints.

**11.** Use according to one of Claims 1 to 8, in which said composition is a cosmetic composition.

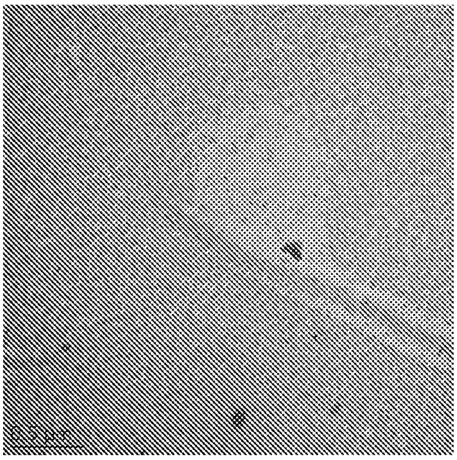

Figure 1

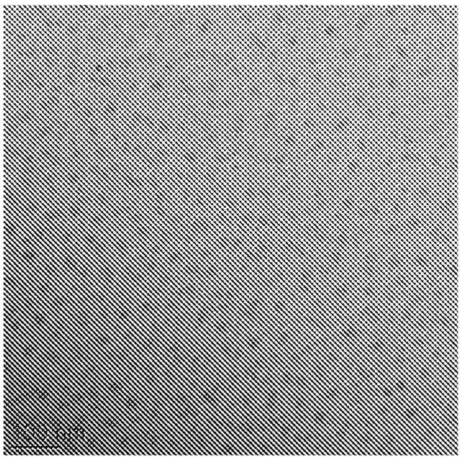

Figure 2

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- WO 2010096867 A


### Littérature non-brevet citée dans la description


- **G. DELAITTRE et al.** *Chem. Commun.,* 2009, 2887-2889
- **S. BOISSÉ et al.** *Chem. Commun.,* 2010, vol. 46, 1950-1952
- **Y. LI ; S.P. ARMES.** *Angew. Chem. Int. Ed.,* 2010, vol. 49, 4042-4046
- **S. SUGIHARA et al.** *Soft Matter,* 2011, vol. 7, 10787